# EUROPEAN PATENT SPECIFICATION

(11) **EP 3 903 748 B1**
(45) Date of publication and mention of the grant of the patent: **10.06.2026**
(21) Application number: 20171928.3
(22) Date of filing: 28.04.2020
(51) Int. Cl.: A61F 7/00, A61F 7/08, B32B 7/12, A61F 7/02

(54) **PORT COVERS FOR FORCED AIR WARMING APPARATUSES**
ANSCHLUSSABDECKUNGEN FÜR ZWANGSLUFTERWÄRMUNGSGERÄTE
COUVERCLES D'ORIFICE POUR APPAREILS DE CHAUFFAGE À AIR FORCÉ

(43) Date of publication of application: 03.11.2021
(73) Proprietor: The Surgical Company International B.V., 1105 AG Amsterdam (NL)
(72) Inventor: TEUNISSEN, Berend Jan, 7481JN Haaksbergen (NL); VAN DE POL, Marcel Elbert-Jan, 3851 WP Ermelo (NL)
(74) Representative: Hindles Limited

(56) References cited:
- WO-A1-2011/014222
- WO-A1-2018/075576
- WO-A1-2019/232056
- US-A1- 2001 012 957
- US-A1- 2014 277 307
- US-A1- 2020 113 730

## Description

### Field of the invention

The present invention relates to the field of temperature-regulation apparatuses including forced air warming apparatuses such as forced air warming blankets.

### Background to the invention

Temperature-regulation apparatuses are used in both hypo- and hyperthermia treatments, as well as before, during and after surgery, to help to regulate or maintain the temperature of a subject. These devices typically take the form of forced air warming devices such as forced air warming blankets.

Forced air warming blankets include air inlet ports through which a supply of temperature regulated air is provided into a chamber or chambers within the blanket and the blanket is thereby inflated. The air is then allowed to slowly leave the chamber(s) via an air permeable wall of the blanket, such that the air is directed towards the patient. The air inlet ports are usually kept closed until a supply of air is required. In some circumstances, it is then preferable to remove the supply of air (optionally without immediately deflating the blanket) and, as such, it is helpful to be able to close the air inlet port. In existing forced air warming blankets, the inlet ports are not reversibly openable and as such, limiting the flow of air out of an inflated blanket, via the inlet port, requires an additional piece of equipment (e.g. a single-use inlet cover) to close the inlet port. If the inlet port then needs to be re-opened and re-closed at a later stage, this necessitates the use of still further inlet covers. Although the inlet cover may be as simple as a sticker, this is inefficient and wasteful of resources. Furthermore, providing inlet covers leads to clutter and presents challenges to a user in efficiently finding inlet covers when they are required during a procedure.

Many blankets have more than one port, as described above. Each port is normally closed and is opened by hand (e.g. by breaking a seal) to connect a means (e.g. a hose) for supplying temperature regulated air into a chamber or chambers within the blanket. In some instances, it is necessary to connect the means (e.g. a hose) for supplying temperature regulated air to another port of the blanket. This leaves the first port open and temperature regulated air can thus escape via this first port. To prevent this, it is common to apply tape to an open port when opening a second port. This creates additional workload for the person attending to a subject whose temperature is to be regulated.

Accordingly, the present invention seeks to provide a forced air warming apparatus which can be used more efficiently, with less additional work for users, and in some embodiments with less waste and requirement for stock components.

US 2020/113730 A1 relates to a closure for releasably sealing an air hose port of inflatable convective thermal blanket. US 2014/0277307 A1 relates to convective air blankets for companion animals, the blankets having inlet ports to allow connection to an air supply from either side of the blanket or an edge of the blanket. WO 2011/014222 A1 relates to an underbody convective warming blanket including a separate duct. WO 2018/075576 A1 relates to a multi-sectional patient warming blanket having an inlet opening couplable to a source of heated air and an outlet opening couplable to the inlet opening of a further warming blanket. WO 2019/232056 A1 relates to an integral inlet port assembly for use as a closable air inlet port for an inflatable heating blanket or garment. US 2001/0012957 A1 relates to a method and apparatus for conveniently and removably sealing inlet ports within an inflatable thermal blanket.

### Summary of the invention

### The invention is set out in the appended set of claims.

A first aspect of the invention provides a forced air warming apparatus having at least two walls which define one or more chambers, each chamber having at least one air permeable wall region and at least one air impermeable wall region, at least two ports for receiving (e.g. temperature-regulated) air into the one or more chambers, each of the ports being formed in a said wall and each port having a port closing panel for covering the port, wherein each port closing panel comprises a frame portion and a closing portion, the closing portion being retained within the frame portion, wherein the closing portion at least partially (and optionally fully) occludes the port thereby restricting the flow of air through the port (to or from one or more chambers), wherein the closing portion can be at least partially removed from the port to allow the flow of air through the port (to or from one or more chambers), wherein the closing portion is partially removable from the port while remaining attached to the frame portion such that the respective port can be used while the closing portion remains attached, wherein the closing portion is removable from the respective closing panel and attachable to a different closing panel, to thereby occlude the port of the different closing panel, and wherein the closing portion comprises at least three closing portion layers, the three closing portion layers comprising a first closing portion layer, an adhesive port-facing closing portion layer, and an adhesive-protecting closing portion release layer.

The closing portions restrict (or prevent) air flow into and out of the one or more chambers via the ports until the apparatus is ready for use. The closing portions typically prevent the attachment of a hose (or conduit, or other means for supplying (e.g. temperature regulated) air) to the respective port until they are (at least partially) removed. In use, a closing portion is partially or fully removed, and a hose or other conduit may then be connected to the respective port to introduce (e.g. temperature regulated) air into one or more chambers. Two or more closing portions may be at least partially removed (or optionally fully removed) at the same or different times, and hoses (or conduits, or other means for supplying (e.g. temperature regulated) air) can then be connected to one or more ports at any given time.

Preferably, the closing portions are removable from the respective closing panels and are attachable to the same or a different closing panel, to thereby occlude the port of the same or a different closing panel (e.g. to restrict or prevent the flow of air into and/or out of a chamber via the said port and/or to prevent the attachment of a conduit). Thus, if when a hose (or other means for supplying temperature regulated air) is disconnected from a first port and air begins to vent through the port, a cover to close the port can be readily found already attached to the apparatus without a requirement to look for a separate cover or tape. The closing portions of at least two or all closing panels may be the same size and shape as each other. This provides the advantage that the closing portions of at least two ports are interchangeable with each other. A closing portion may be removed from one port and attached to another port. This means that if for example a hose is to be removed from a first port and attached to a second port then the closing portion from the second port may be removed and attached to the first port. This means that a closing portion to close the first port is immediately available (attached to the second port) without a requirement to stock separate closing portions, which may not be readily to hand in a hospital environment.

The closing portion is (e.g. at least partially, optionally completely) removable from the port. The closing portion is partially removable while remaining attached to the frame portion such that the respective port can be used while the closing portion remains attached (for example, a hose can be attached, and (e.g. temperature regulated) air can be thereby introduced into one or more chambers through the port). It may be that the closing portion can then be (e.g. fully) attached to the port again, optionally without having been fully detached. Because the closing portion remains attached, it will not be misplaced and is readily available to close the port again, for example when a hose is removed again.

In some embodiments, the closing portion is partially and/or fully removable from the port. A user may therefore choose whether to partially or fully remove the closing portion. For example, the closing portion may be partially detachable to open the port but remains attached, for example through a connection, and also be fully detachable, for example through a breakable (e.g. tearable) connection. This is useful as the closing portion can be partially removed to enable the port to be used, without the closing portion becoming separated from the forced air warming apparatus, but can then be detached and fully removed in order to attach it to either another port or to the same port.

It may be that the port closing panels are planar. Typically, the frame portion is a planar, annular frame portion, however in some embodiments the frame portion may have another shape, for example it may or may not extend entirely around the closing portion. The frame portion may be circular or another shape, for example, square (or triangular, rectangular, hexagonal, oval, etc). The closing portion may be circular, or another shape, for example, where the frame portion is square in shape, the closing portion may be correspondingly square in shape. Typically, the shape of the closing portion corresponds to the shape of the frame portion. Planar port closing panels enable the forced air warming apparatus to take up less space when being stored (i.e. before use and before being inflated).

Typically, the perimeter of the closing portion is at least partially defined by a dividing line between the closing portion and the surrounding frame portion, such that the closing portion can be at least partially removed from the closing portion. The dividing line, between the closing portion and the surrounding frame portion, may be a cut-line, or a line of weakness or a line of perforations. The dividing line may extend partially but not entirely around the periphery of the closing portion. In this case, the closing panel typically comprises a hinge portion, the hinge portion connecting the closing portion and the frame portion. The hinge portion may be foldable such that the closing portion can be (typically reversibly) folded away from the frame portion via the foldable hinge portion to allow use of the port whilst the closing portion remains connected to the closing panel. The hinge can be flexed (e.g. folded back) to partially remove the closing panel from the port. It may be that the hinge portion is tearable and the closing portion is tearably attached to (and/or is tearably removable from) the frame portion.

This provides the advantage that the closing portion can be partially (optionally reversibly) moved away from the frame portion (i.e. in use) to thereby allow (e.g. temperature regulated) air to flow through the port (for example to allow a hose to be attached to the port), while remaining attached. It may then be repositioned to occlude the port or in some embodiments removed (e.g. torn) and used to occlude the same or another port.

The closing portion comprises at least three closing portion layers, the three closing portion layers comprising a first closing portion layer, an adhesive port-facing closing portion layer, and an adhesive-protecting closing portion release layer. Typically, the first closing portion layer is formed of a paper or plastics material, e.g. card.

Typically, each port closing panel is the same size and shape.

The forced air warming apparatus comprises one or more chambers and at least two ports. The ports are in the chamber walls. The ports extend from within a chamber to the outside of the forced air warming apparatus. It may be that at least two ports are in the walls of the same chamber. It may be that the forced air warming apparatus has one chamber with two ports (or more). However, it may be that the forced air warming apparatus comprises two (or more) separate chambers, each of which has a (e.g. single) port.

The forced air warming apparatus may comprise two sheets of flexible material which are joined to each other by connections. The one or more chambers may be defined by connections between the sheets. The connections may be welds. It may be that one sheet is air permeable and the other sheet is air impermeable. The sheets may comprise a plurality of layers.

Typically, at least some of the material which forms the wall of the apparatus on which a port is located extends within the frame portion. Thus, there is material (wall material) within the frame portion to which a closing portion may be attached, for example adhered. The said material may be an air permeable sheet or an air impermeable sheet. Typically, each port is formed in one of the walls and/or the material by one or more slits, for example two slits which cross each other.

Typically, each closing portion is sized and/or shaped to cover the ports. Each closing portion may be larger (e.g. in area) than the ports.

Typically, the port closing panels each comprise connectors for conduits, such as hoses. The frame portion may function as the connector. It may be that a closing portion must be at least partially removed from the frame portion to enable a conduit to be connected to the frame portion. The frame portion may have an inner edge and comprise a plurality of cut-lines extending from the inner edge, e.g. radially outwards. These enable a hose or other conduit to connect to the frame portion to enable temperature-regulated air to be introduced into at least one chamber through the port.

According to a second aspect of the invention there is provided a port closing panel for a forced air warming apparatus, the port closing panel comprising a frame portion and a closing portion, the closing portion being retained within the frame portion, wherein the closing portion at least partially (and optionally fully) occludes a port in use to thereby restrict the flow of air through the port, and wherein the closing portion can be (e.g. at least partially) removed from the closing panel to allow the flow of air through a port to which the port closing panel is fitted in use. The forced air warming apparatus has at least two ports and each port has a port closing panel for covering said port. The closing portion is partially removable from the port while remaining attached to the frame portion such that the respective port can be used while the closing portion remains attached. The closing portion is removable from the respective closing panel and attachable to a different closing panel, to thereby occlude the port of the different closing panel. The closing portion comprises at least three closing portion layers, the three closing portion layers comprising a first closing portion layer, an adhesive port-facing closing portion layer, and an adhesive-protecting closing portion release layer.

A third aspect of the invention provides a kit of parts comprising a forced air warming apparatus having at least two walls which define one or more chambers, each chamber having at least one air permeable wall region and at least one air impermeable wall region, at least two ports for receiving (e.g. temperature-regulated) air into the one or more chambers, each of the ports being formed in a said wall and a plurality of port closing panels according to the second aspect of the invention.

A fourth aspect of the invention provides a method of operating a forced air warming apparatus according to the first aspect of the invention, the method comprising the step of at least partially removing a first closing portion from the port closing panel of a first port, connecting an air supply conduit (e.g. a hose) to the first port (typically via the frame portion), introducing (e.g. temperature-regulated) air through the port into the chamber to inflate the chamber and subsequently removing the supply of air, detaching a second closing portion from the port closing panel of a second port and attaching the first closing portion to the second port (whether directly or to the port closing panel of the second port) to thereby at least partially occlude the second port. The method may comprise removing an adhesive protecting release layer from the first closing portion to reveal an adhesive layer and using the adhesive layer to attach the first closing portion to the second port. The walls are typically formed of fabric. It may be that the first closing portion is adhered to fabric within the frame portion of the second port closing panel.

The invention extends in a fifth aspect to a method of making a forced air warming apparatus comprising bringing a first sheet of an air-impermeable material and a second sheet of an air-permeable material into contact; forming one or more seals between the first sheet and the second sheet to define one or more chambers therein; forming at least two ports in the first sheet or in the second sheet; and attaching one or more port closing panels (i.e. port closing panels according to the second aspect of the invention) to the first sheet or the second sheet such that the or each port closing panel covers the or each port. The steps may be carried out in various orders. The ports may be formed by cutting slits in the first and/or second sheet, whether before or after they are joined to each other. The resulting forced air warming apparatus is typically a forced air warming apparatus according to the first aspect of the invention.

Although various aspects and embodiments of the present invention have been described separately above, any of the aspects and features of the present invention can be used in conjunction with any other aspect, embodiment or feature where appropriate. For example, apparatus features may where appropriate be interchanged with method features. References to single entities should, where appropriate, be considered generally applicable to multiple entities and vice versa. Unless otherwise stated herein, no feature described herein should be considered to be incompatible with any other, unless such a combination is clearly and inherently incompatible. Accordingly, it should generally be envisaged that each and every separate feature disclosed in the introduction, description and drawings is combinable in any appropriate way with any other unless (as noted above) explicitly or clearly incompatible.

### Description of the drawings

Figure 1 is a plan view of a forced air warming blanket with two air inlet ports according to a first example embodiment of the invention;
Figure 2 is a cross-sectional view of a forced air warming blanket along lines A-A of figure 1, with two chambers and two air inlet ports as inflated;
Figure 3a is a plan view of a port closing panel according to a first example embodiment of the invention;
Figure 3b is a plan view of a port closing panel with the closing portion removed and a wall of the forced air warming blanket exposed;
Figure 3c is a perspective view of a port closing panel with the closing portion folded away from the frame portion;
Figure 4 is a cross-sectional view of the closing portion of a port closing panel; and
Figure 5 is a cross-sectional view of a closing sheet, not according to the invention.

### Detailed description of an example embodiment

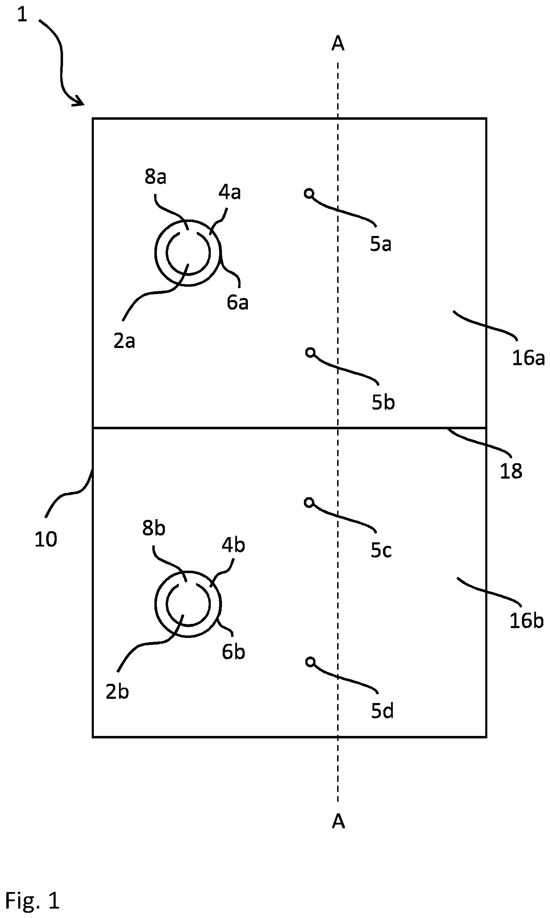

With reference to Figure 1, a temperature regulation apparatus according to the invention takes the form of a forced air warming blanket 1 having two fabric walls which are joined to each other to define two chambers 16a, 16b. Of the walls, one is an air permeable wall 14 and one is an air impermeable wall 12. The blanket 1 also has ports (i.e. inlet ports) for receiving air into the chambers 16, said ports being formed in the walls. The two chambers 16a, 16b are separated by an interior weld 18. The external edge of the blanket is formed by an external heat seal 10.

Each inlet port has a port closing panel 6. The port closing panels 6 allow their respective port to be closed to limit the flow of air into or out of a chamber 16. The port closing panels 6 each have a frame portion 4 which surrounds the inlet port and which is adhered to the fabric, and a closing portion 2, which is positioned within the frame portion 4 and which occludes the port. The frame portion 4 and the closing portion 2 are connected by a foldable hinge portion 8. Accordingly, during use, the closing portion 2 can be folded away from the frame portion 4 to open the inlet port and to allow the connection of a hose to introduce of a supply air into a chamber 16 to thereby inflate the chamber 16.

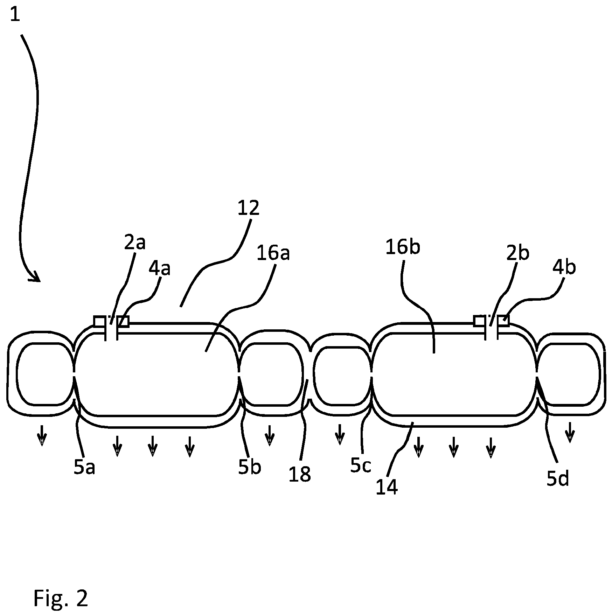

Figure 2 is a cross-sectional view through the forced air warming blanket 1 when the two chambers 16a, 16b are inflated. Each chamber has internal welds 5a, 5b, 5c, 5d which prevent the chambers from ballooning excessively and which define the shape of the blanket when inflated and the way in which air flows through the blanket. Hoses (not shown) are connected to the ports to inflate the chambers and force (e.g. temperature regulated) pressurised air through the air permeable sheet to warm a subject.

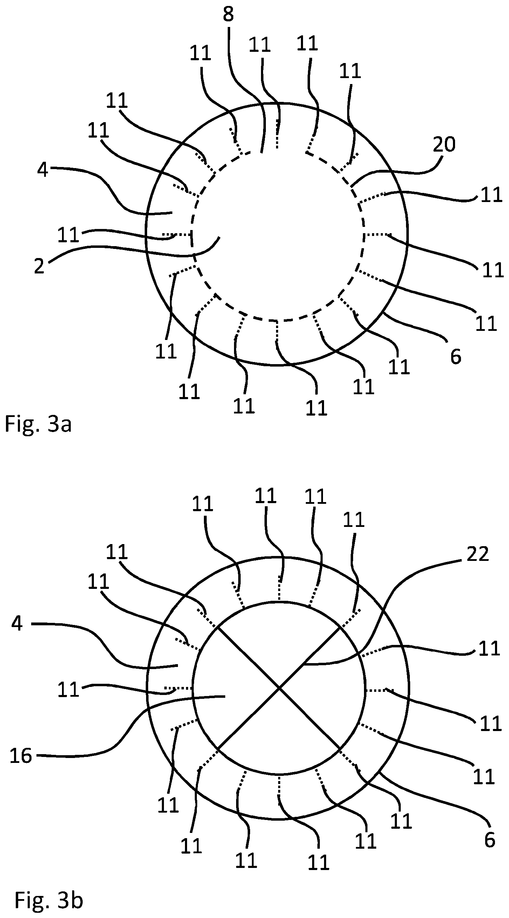

Figures 3a and 3b are plan views of port closing panels 6. In this example embodiment, the perimeter closing portion 2 of a port closing panel 6 is formed in the port closing panel 6 by a dividing line in the form of a cut-line 20 which separates the closing portion 2 from the frame portion 4. The perimeter of the closing portion is an incomplete perimeter (i.e. the dividing line does not form a complete circle), which provides for the foldable hinge portion 8 which connects the closing portion 2 to the frame portion 4.

The frame portion 4 of a port closing panel 6 has a series of tab-defining cut-lines 11, being cut radially outwards from the centre of the port, to thereby create a series of tabs in the inner part of the frame portion 4. These tabs then form a port connector for the attachment of a hose which can supply (e.g. temperature-regulated) air into a chamber 16 in use. The air can then slowly leave the chamber 16 via the air permeable wall 14.

Each inlet port is formed by two slits 22 which bisect each other to form a cross, the slits 22 being cut in one of the walls of the blanket (most typically an air impermeable wall 12 but the person skilled in the art will appreciate that in some embodiments it would be possible to form the slits 22 and hence the inlet port in the air permeable wall 14). The slits 22 allow air to flow freely into or out of the chamber 16 in the absence of a port closing panel 6 or other similar object positioned over the slits 22. Each port closing panel 6 surrounds an inlet port and each closing portion 2 covers and occludes an inlet port. Accordingly, each port closing panel 6 also surrounds an area of a wall 24 in which the slits 22 are cut.

One skilled in the art will appreciate that there may be other ways of forming an inlet port which are similarly suitable. For example, an inlet port might be formed a single slit, or from a plurality of slits, or from slits that do not bisect to form a cross. Alternatively, an inlet port may be formed by a mesh or grid section within a wall, or, in the case that an inlet port is formed in the air impermeable wall 14 (as is typically the case) the said inlet port may be formed by an air permeable section within the air impermeable wall 14. A further alternative would be to provide an aperture in a wall to thereby provide an inlet port. Typically, however, there is some area of a wall which is circumscribed by the frame portion 4 of an inlet closing panel 6 and, as such, even where an inlet port is formed by an aperture in the wall, the aperture is smaller (i.e. in area) than the area of wall that is circumscribed by the frame portion 4.

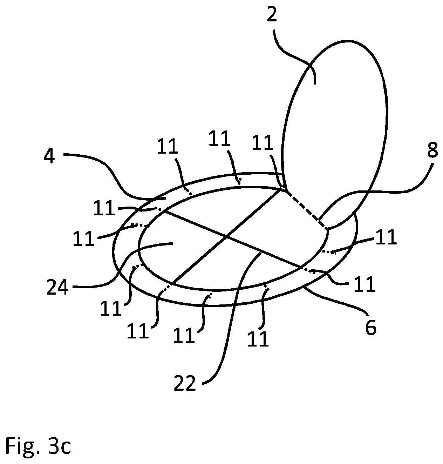

Figure 3c is a perspective view of a port closing panel 6 wherein the closing portion 8 has been folded away from the frame portion 4 via the foldable hinge portion 8. In figure 3c, the port closing panel has been partially removed from the port to thereby allow access to the port.

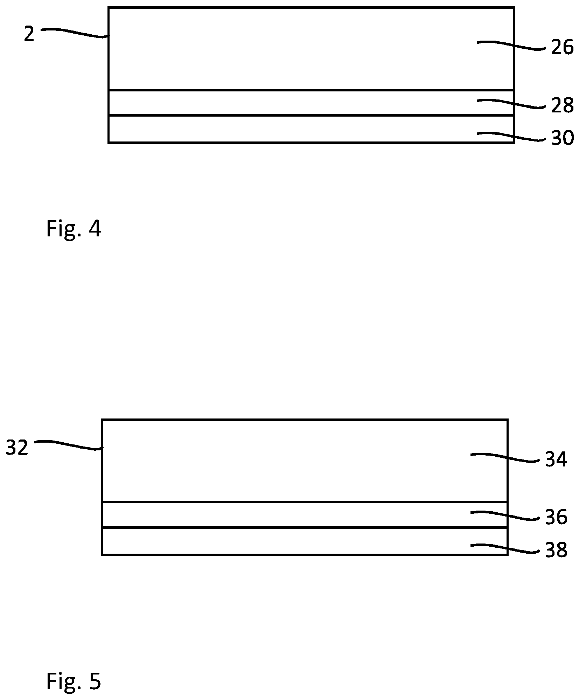

Turning to Figure 4, which is a cross-sectional view through a closing portion 2 of a port closing panel 6. The closing portion 2 is formed of three layers; a first closing portion layer 26, an adhesive closing portion layer 28 and an adhesive-protecting closing portion release layer 30. Accordingly, when the closing portion has been folded away from the frame portion 4 (as shown in figure 3c), it is possible to subsequently remove the adhesive-protecting closing release layer 30 from the closing portion 2, to fold the closing portion 8 back towards the frame portion 4, and to attach the adhesive closing portion layer 28 to the area of wall that is circumscribed by the frame portion 4, to thereby re-occlude the inlet port.

The foldable hinge portion 8 is generally tearable. This allows the closing portion 2 to be torn away from and thereby removed from the frame portion 8. This might be preferable if it allows a supply of air to be introduced into a chamber more easily, for example if the closing portion 2 is in the way, making it difficult for a user to connect, for example, a hose, for the supply of air. Subsequently, for example where the chamber 16 is inflated and no further air supply is required, it is possible for a user to disconnect the air supply, remove the adhesive-protecting closing portion release layer 30 from the adhesive closing portion layer 28 of the closing portion 2, and attach the closing portion 2 to the port (i.e. to the area of wall circumscribed by the frame portion 4 of the port closing panel 6) via the closing portion adhesive layer 28, to thereby occlude the port and re-limit the flow of air into or out of the chamber 16.

The closing portions 2 of each port closing panel 6 are interchangeable with each other in that, if a closing portion 2 is removed from a first port closing panel 6, the adhesive-protecting closing portion release layer 30 can be removed from the said closing portion and the closing portion 2 (i.e. the adhesive closing portion layer 28 of the closing portion 2) can then either be attached to the port closing panel 6 of the same port (i.e. to the area of wall circumscribed by the frame portion 4 of the port closing panel 6), or to the port closing panel 6 of a different port (i.e. to an area of wall circumscribed by a frame portion 4 of a different port closing panel 6). Furthermore, due to the presence of a tearable hinge, a closing portion 2 may be folded back to partially remove it from the port which it occludes, while it remains attached. At a later time, it can be torn off and used to close another port.

In one application, the forced air warming blanket 1 is placed over the body of a subject with the air permeable wall 14 underneath, facing the subject. The head of the subject extends from one end of the forced air warming blanket 1, and the body of the subject is substantially covered by the forced air warming blanket 1.

This configuration of a forced air warming blanket 1 allows the active control of the temperature of a subject by regulating the temperature of the air which is supplied into each of the chambers 16 and which is subsequently forced through the air permeable wall 14 which is generally positioned such that air leaving a chamber 16 via the air permeable wall 14 is directed towards the said subject, as described above. This is advantageous in the treatment of hypo- or hyperthermia.

During use, it may be that one chamber is inflated by attaching an air blower to its port and then, later, the other chamber may be inflated instead through its port. Sometimes both chambers may be used at once, possibly with air of different temperature. Either the air permeable wall 14 or the air impermeable wall 12 may have more than one layer. For example, the air impermeable wall may comprise a non-fabric layer and a metallic reflecting layer between first and second layers. The air permeable wall may comprise an inner layer and an outer layer, where the inner layer has a greater resistance to the flow of air to thereby distribute air across the air permeable wall.

In some applications, not according to the invention, the apparatus is used with a plurality of separate closing sheets 32 (see Figure 5, which is a cross-sectional view through a closing sheet 32), which may conveniently be supplied in the form of adhesive stickers (e.g. on a roll or a sheet). Said closing sheets 32 may be used interchangeably with a closing portion 2 of a closing panel 6. For example, if a closing portion 2 is removed from a closing panel 6 (e.g. by tearing the foldable hinged portion 8) and is then lost or damaged, a user of the forced air warming blanket may instead close the port by applying a closing sheet 32.

Such closing sheets 32 may have just two layers, a first closing sheet layer 34, and an adhesive layer 36 which may be adhered to a roll of tape which functions as a release paper. Accordingly, where the chamber 16 is inflated and no further air supply is required, it is possible for a user to disconnect the air supply and attach a closing sheet 32 to the port (i.e. to the area of wall circumscribed by the frame portion 4 of the port closing panel 6) via the closing portion adhesive layer 36, to thereby occlude the port and re-limit the flow of air into or out of the chamber 16.

In a further alternative application, the forced air warming blanket 1 may be used under the subject (e.g. as a forced air underbody). That is, the subject lies on the forced air warming blanket 1 (i.e. on the air permeable wall 14 of the forced air warming blanket 1) and (e.g. temperature regulated) air is conveyed to the underside of the subject. In this case, the air impermeable layer is arranged away from the subject and the air permeable layer is arranged against (i.e. in contact with) the subject. Accordingly, the forced air warming blanket 1 of the invention may be used either above or below a subject, or two forced air warming blankets may be used simultaneously, with one arranged above and one arranged below a subject.

During manufacture, the forced air warming blanket 1 of the present example is formed by the connection of the first (air permeable) wall 14 and second (air impermeable) wall 12 to each other by an external heat seal 10, internal spot welds 5a, 5b, 5c, 5d and the interior weld 18. This thereby forms a forced air warming blanket 1 having first and second chambers 16a, 16b. The skilled person will appreciate that other configurations may be suitable, for example with additional interior welds where additional chambers are required. The manufacturing process may be a reel-to-reel manufacturing process, wherein multiple forced air warming blankets are produced sequentially.

Although in the example shown in Figure 2 the forced air warming blanket 1 has two inflatable chambers 16, in further example embodiments, the forced air warming blanket 1 may have one inflatable chamber 16 or three inflatable chambers 16 or a plurality of inflatable chambers 16. Likewise, although in the example shown in Figure 2 the inflatable chambers 16 of the forced air warming blanket 1 may be substantially the same size (and/or shape) in other examples each inflatable chamber 16 may be a different size (and/or shape) from the or each other inflatable chamber 16.

Likewise, although in the example shown in Figure 2 both inflatable chambers 16 of the forced air warming blanket 1 are inflated (and this is a typical use scenario), in operation it would be common to inflate one inflatable chamber 16 only (for example, where only one part of the body of the subject should be supplied with temperature conditioned or temperature regulated air). It is also possible to supply temperature conditioned or temperature regulated gas to one chamber 16 which is a different temperature to that supplied to another chamber 16.

In a further example embodiment, the perimeter of the closing portion 2 of a port closing panel 6 might be a dividing-line formed by a line of perforations or a line of weakness (i.e. instead of by a cut-line). In this case, the dividing-line may be continuous to thereby allow a user to determine whether a hinge portion 8 is needed or preferred and, if so, where exactly around the perimeter of the closing portion 2 the hinge portion 8 should be located.

Further variations and modifications may be made within the scope of the invention herein disclosed.

List of reference numerals used in the drawings:
1 = forced air warming apparatus;
2, 2a, 2b = closing portion of port closing panel;
4, 4a, 4b = frame portion of port closing panel;
5a, 5b = internal welds;
6, 6a, 6b = port closing panel;
8, 8a, 8b = foldable hinge portion;
10 = external heat seal;
11 = tab-defining cut-line;
12 = first wall;
14 = second wall;
16a = first chamber;
16b = second chamber;
18 = interior weld;
20 = cut line/line of weakness/line of perforations;
22 = slit;
24 = wall circumscribed within outer portion of port closing panel;
26 = first layer of closing portion;
28 = adhesive layer of closing portion;
30 = release layer of closing portion;
32 = closing sheet;
34 = first layer of closing sheet;
36 = adhesive layer of closing sheet;
38 = release layer of closing sheet.

## Claims

1. A forced air warming apparatus (1) having at least two walls (12, 14) which define one or more chambers (16a, 16b), each chamber having at least one air permeable wall region and at least one air impermeable wall region, at least two ports for receiving air into the one or more chambers, each of the ports being formed in a said wall, wherein each port has a port closing panel (6, 6a, 6b) for covering the port, wherein each port closing panel comprises a frame portion (4, 4a, 4b) and a closing portion (2, 2a, 2b), the closing portion being retained within the frame portion, wherein the closing portion at least partially occludes the port thereby restricting the flow of air through the port, wherein the closing portion can be at least partially removed from the port to allow the flow of air through the port,
wherein the closing portion (2, 2a, 2b) is partially removable from the port while remaining attached to the frame portion (4, 4a, 4b) such that the respective port can be used while the closing portion remains attached, **characterized in that** the closing portion (2, 2a, 2b) is removable from the respective closing panel and attachable to a different closing panel, to thereby occlude the port of the different closing panel, and
wherein the closing portion (2, 2a, 2b) comprises at least three closing portion layers, the three closing portion layers comprising a first closing portion layer (26), an adhesive port-facing closing portion layer (28), and an adhesive-protecting closing portion release layer (30).

2. A forced air warming apparatus (1) according to claim 1, wherein the closing portions (2, 2a, 2b) are removable from the respective closing panels (6, 6a, 6b) and attachable to the same closing panel, to thereby occlude the port of the same closing panel.

3. A forced air warming apparatus (1) according to claim 1 or claim 2, wherein the closing portions (2, 2a, 2b) of at least two or all closing panels are the same size and shape as each other.

4. A forced air warming apparatus (1) according to any one preceding claim, wherein the closing portion (2, 2a, 2b) is partially detachable to open the port but remains attached, for example through a breakable connection.

5. A forced air warming apparatus (1) according to any one preceding claim, wherein the port closing panels (6, 6a, 6b) are planar.

6. A forced air warming apparatus (1) according to any one preceding claim, wherein the perimeter of the closing portion (2, 2a, 2b) is at least partially defined by a dividing line between the closing portion and the surrounding frame portion (4, 4a, 4b), such that the closing portion can be at least partially removed from the frame portion, optionally wherein the dividing line is a cut-line, or a line of weakness or a line of perforations.

7. A forced air warming apparatus (1) according to claim 6, wherein the dividing line extends partially but not entirely around the periphery of the closing portion (2, 2a, 2b) and the closing panel (6, 6a, 6b) comprises a hinge portion (8, 8a, 8b) , the hinge portion connecting the closing portion and the frame portion.

8. A forced air warming apparatus (1) according to claim 7, wherein the hinge portion (8, 8a, 8) is foldable such that the closing portion (2, 2a, 2b) can be folded away from the frame portion (4, 4a, 4b) via the foldable hinge portion to allow use of the port whilst the closing portion remains connected to the closing panel, optionally wherein the hinge portion is tearable and the closing portion is tearably attached to and/or is tearably removable from the frame portion.

9. A forced air warming apparatus (1) according to any one preceding claim, wherein at least some of the material which forms a wall of the forced air apparatus (1) extends within the frame portion (4, 4a, 4b).

10. A forced air warming apparatus (1) according to any one preceding claim, wherein the forced air warming apparatus comprises one chamber (16a, 16b) with two ports, or two or more separate chambers, each of which has a port.

11. A forced air warming apparatus (1) according to any one preceding claim which comprises two sheets of flexible material which are joined to each other by connections (5a, 5b, 10), wherein the one or more chambers (16a, 16b) may be defined by connections between the sheets, wherein one sheet is air permeable and the other sheet is air impermeable, and at least some of the material which forms the wall of the apparatus on which a port is located extends within the frame portion (4, 4a, 4b).

12. A method of making a forced air warming apparatus (1) comprising bringing a first sheet (12) of an air-impermeable material and a second sheet of an air-permeable material into contact (14); forming one or more seals (51, 5b, 10) between the first sheet and the second sheet to define one or more chambers (16a, 16b) therein; forming at least two ports in the first sheet or in the second sheet; and attaching one or more port closing panels (6, 6a, 6b) to the first sheet or the second sheet such that the or each port closing panel covers the or each port,
wherein each port closing panel (6, 6a, 6b) comprises a frame portion (4, 4a, 4b) and a closing portion (2, 2a, 2b), the closing portion (2, 2a, 2b) being retained within the frame portion (4, 4a, 4b), wherein the closing portion (2, 2a, 2b) at least partially occludes the port thereby restricting the flow of air through the port, wherein the closing portion (2, 2a, 2b) can be at least partially removed from the port to allow the flow of air through the port, wherein the closing portion (2, 2a, 2b) is partially removable from the port while remaining attached to the frame portion (4, 4a, 4b) such that the respective port can be used while the closing portion remains attached,
**characterized in that** the closing portion (2, 2a, 2b) is removable from the respective closing panel and attachable to a different closing panel, to thereby occlude the port of the different closing panel, and
wherein the closing portion (2, 2a, 2b) comprises at least three closing portion layers, the three closing portion layers comprising a first closing portion layer (26), an adhesive port-facing closing portion layer (28), and an adhesive-protecting closing portion release layer (30).

## Patentansprüche

1. Zwangslufterwärmungseinrichtung (1) mit mindestens zwei Wänden (12,14), die eine oder mehrere Kammern (16a, 16b) definieren, wobei jede Kammer mindestens einen luftdurchlässigen Wandbereich und mindestens einen luftundurchlässigen Wandbereich, mindestens zwei Anschlüsse zum Aufnehmen von Luft in die eine oder mehreren Kammern, aufweist, wobei jeder der Anschlüsse in einer der Wände ausgebildet ist, wobei jeder Anschluss ein Anschlussverschlusspaneel (6, 6a, 6b) zum Abdecken des Anschlusses aufweist, wobei jedes Anschlussverschlusspaneel einen Rahmenabschnitt (4, 4a, 4b) und einen Verschlussabschnitt (2, 2a, 2b) umfasst, wobei der Verschlussabschnitt innerhalb des Rahmenabschnitts gehalten wird, wobei der Verschlussabschnitt den Anschluss mindestens teilweise verschließt, wodurch der Luftstrom durch den Anschluss eingeschränkt wird, wobei der Verschlussabschnitt mindestens teilweise von dem Anschluss entfernt werden kann, um den Luftstrom durch den Anschluss zu ermöglichen, wobei der Verschlussabschnitt (2, 2a, 2b) teilweise von dem Anschluss entfernbar ist, während er am Rahmenabschnitt (4, 4a, 4b) befestigt bleibt, so dass der jeweilige Anschluss verwendet werden kann, während der Verschlussabschnitt befestigt bleibt, **dadurch gekennzeichnet, dass** der Verschlussabschnitt (2, 2a, 2b) von dem jeweiligen Verschlusspaneel entfernbar und an einem anderen Verschlusspaneel befestigbar ist, um dadurch den Anschluss des anderen Verschlusspaneels zu verschließen, und
wobei der Verschlussabschnitt (2, 2a, 2b) mindestens drei Verschlussabschnittschichten umfasst, wobei die drei Verschlussabschnittschichten eine erste Verschlussabschnittschicht (26), eine dem Anschluss zugewandte Verschlussabschnittklebeschicht (28) und eine klebstoffschützende Verschlussabschnitttrennschicht (30) umfassen.

2. Zwangslufterwärmungseinrichtung (1) nach Anspruch 1, wobei die Verschlussabschnitte (2, 2a, 2b) von den jeweiligen Verschlusspaneelen (6, 6a, 6b) entfernbar und an demselben Verschlusspaneel befestigbar sind, um dadurch den Anschluss desselben Verschlusspaneels zu verschließen.

3. Zwangslufterwärmungseinrichtung (1) nach Anspruch 1 oder Anspruch 2, wobei die Verschlussabschnitte (2, 2a, 2b) von mindestens zwei oder allen Verschlusspaneelen dieselbe Größe und Form wie die anderen aufweisen.

4. Zwangslufterwärmungseinrichtung (1) nach einem der vorstehenden Ansprüche, wobei der Verschlussabschnitt (2, 2a, 2b) teilweise entfernbar ist, um den Anschluss zu öffnen, aber befestigt bleibt, beispielsweise durch eine brechbare Verbindung.

5. Zwangslufterwärmungseinrichtung (1) nach einem der vorstehenden Ansprüche, wobei die Anschlussverschlusspaneele (6, 6a, 6b) planar sind.

6. Zwangslufterwärmungseinrichtung (1) nach einem der vorstehenden Ansprüche, wobei der Umfang des Verschlussabschnitts (2, 2a, 2b) mindestens teilweise durch eine Trennlinie zwischen dem Verschlussabschnitt und dem umgebenden Rahmenabschnitt (4, 4a, 4b) definiert ist, so dass der Verschlussabschnitt mindestens teilweise von dem Rahmenabschnitt entfernt werden kann, wobei optional die Trennlinie eine Schnittlinie oder eine Schwächungslinie oder eine Perforationslinie ist.

7. Zwangslufterwärmungseinrichtung (1) nach Anspruch 6, wobei sich die Trennlinie teilweise, aber nicht vollständig um den Umfang des Verschlussabschnitts (2, 2a, 2b) erstreckt und das Verschlusspaneel (6, 6a, 6b) einen Scharnierabschnitt (8, 8a, 8b) umfasst, wobei der Scharnierabschnitt den Verschlussabschnitt und den Rahmenabschnitt verbindet.

8. Zwangslufterwärmungseinrichtung (1) nach Anspruch 7, wobei der Scharnierabschnitt (8, 8a, 8) klappbar ist, so dass der Verschlussbereich (2, 2a, 2b) über den klappbaren Scharnierabschnitt von dem Rahmenabschnitt (4, 4a, 4b) weggeklappt werden kann, um die Verwendung des Anschlusses zu ermöglichen, während der Verschlussabschnitt mit dem Verschlusspaneel verbunden bleibt, wobei optional der Scharnierabschnitt abreißbar ist und der Verschlussabschnitt abreißbar an dem Rahmenbereich befestigt ist und/oder abreißbar von dem Rahmenbereich entfernbar ist.

9. Zwangslufterwärmungseinrichtung (1) nach einem der vorstehenden Ansprüche, wobei sich mindestens ein Teil des Materials, das eine Wand der Zwangslufteinrichtung (1) bildet, innerhalb des Rahmenabschnitts (4, 4a, 4b) erstreckt.

10. Zwangslufterwärmungseinrichtung (1) nach einem der vorstehenden Ansprüche, wobei die Zwangslufterwärmungseinrichtung eine Kammer (16a, 16b) mit zwei Anschlüssen oder zwei oder mehr separate Kammern umfasst, von denen jede einen Anschluss aufweist.

11. Zwangslufterwärmungseinrichtung (1) nach einem der vorstehenden Ansprüche, umfassend zwei Bahnen aus flexiblem Material, die durch Verbindungen (5a, 5b, 10) miteinander verbunden sind, wobei die eine oder mehreren Kammern (16a, 16b) durch Verbindungen zwischen den Bahnen definiert sein können, wobei eine Bahn luftdurchlässig und die andere Bahn luftundurchlässig ist, und mindestens ein Teil des Materials, das die Wand der Vorrichtung bildet, an der sich ein Anschluss befindet, sich innerhalb des Rahmenabschnitts (4, 4a, 4b) erstreckt.

12. Verfahren zum Herstellen einer Zwangslufterwärmungseinrichtung (1), umfassend das Inkontaktbringen (14) einer ersten Bahn (12) aus einem luftundurchlässigen Material und einer zweiten Bahn aus einem luftdurchlässigen Material; Bilden einer oder mehrerer Dichtungen (51, 5b, 10) zwischen der ersten Bahn und der zweiten Bahn, um eine oder mehrere Kammern (16a, 16b) darin zu definieren; Ausbilden von mindestens zwei Anschlüssen in der ersten Bahn oder in der zweiten Bahn; und Befestigen einer oder mehrerer Anschlussverschlusspaneele (6, 6a, 6b) an der ersten Bahn oder der zweiten Bahn, so dass das oder jedes Anschlussverschlusspaneel den oder jeden Anschluss abdeckt, wobei jedes Anschlussverschlusspaneel (6, 6a, 6b) einen Rahmenabschnitt (4, 4a, 4b) und einen Verschlussabschnitt (2, 2a, 2b) umfasst, wobei der Verschlussabschnitt (2, 2a, 2b) innerhalb des Rahmenabschnitts (4, 4a, 4b) gehalten wird, wobei der Verschlussabschnitt (2, 2a, 2b) den Anschluss mindestens teilweise verschließt, wodurch der Luftstrom durch den Anschluss eingeschränkt wird, wobei der Verschlussabschnitt (2, 2a, 2b) mindestens teilweise von dem Anschluss entfernt werden kann, um den Luftstrom durch den Anschluss zu ermöglichen, wobei der Verschlussabschnitt (2, 2a, 2b) teilweise von dem Anschluss entfernbar ist, während er an dem Rahmenabschnitt (4, 4a, 4b) befestigt bleibt, so dass der jeweilige Anschluss verwendet werden kann, während der Verschlussabschnitt befestigt bleibt, **dadurch gekennzeichnet, dass** der Verschlussabschnitt (2, 2a, 2b) von dem jeweiligen Verschlusspaneel entfernbar und an einem anderen Verschlusspaneel befestigbar ist, um dadurch den Anschluss des anderen Verschlusspaneels zu verschließen, und
wobei der Verschlussabschnitt (2, 2a, 2b) mindestens drei Verschlussabschnittschichten umfasst, wobei die drei Verschlussabschnittschichten eine erste Verschlussabschnittschicht (26), eine dem Anschluss zugewandte Verschlussabschnittklebeschicht (28) und eine klebstoffschützende Verschlussabschnitttrennschicht (30) umfassen.

## Revendications

1. Appareil de réchauffement d'air forcé (1) ayant au moins deux parois (12, 14) qui définissent une ou des chambres (16a, 16b), chaque chambre ayant au moins une région de paroi perméable à l'air et au moins une région de paroi imperméable à l'air, au moins deux orifices permettant de recevoir de l'air dans la ou les chambres, chacun des orifices étant formé dans une dite paroi, dans lequel chaque orifice a un panneau de fermeture d'orifice (6, 6a, 6b) pour couvrir l'orifice, dans lequel chaque panneau de fermeture d'orifice comprend une partie de cadre (4, 4a, 4b) et une partie de fermeture (2, 2a, 2b), la partie de fermeture étant retenue à l'intérieur de la partie de cadre, dans lequel la partie de fermeture obstrue au moins partiellement l'orifice limitant ainsi la circulation d'air à travers l'orifice, dans lequel la partie de fermeture peut être au moins partiellement retirée de l'orifice pour permettre l'écoulement d'air à travers l'orifice, dans lequel la partie de fermeture (2, 2a, 2b) est partiellement amovible de l'orifice tout en restant fixée à la partie cadre (4, 4a, 4b) de telle sorte que l'orifice respectif peut être utilisé tandis que la partie de fermeture reste fixée, **caractérisé en ce que** la partie de fermeture (2, 2a, 2b) peut être retirée du panneau de fermeture respectif et peut être fixée à un panneau de fermeture différent, afin d'obstruer ainsi l'orifice du panneau de fermeture différent, et
dans lequel la partie de fermeture (2, 2a, 2b) comprend au moins trois couches de partie de fermeture, les trois couches de partie de fermeture comprenant une première couche de partie de fermeture (26), une couche de partie de fermeture adhésive orientée vers l'orifice (28) et une couche de libération de partie de fermeture protectrice adhésive (30).

2. Appareil de réchauffement d'air forcé (1) selon la revendication 1, dans lequel les parties de fermeture (2, 2a, 2b) peuvent être retirées des panneaux de fermeture respectifs (6, 6a, 6b) et fixées au même panneau de fermeture, afin d'obstruer ainsi l'orifice du même panneau de fermeture.

3. Appareil de réchauffement d'air forcé (1) selon la revendication 1 ou la revendication 2, dans lequel les parties de fermeture (2, 2a, 2b) d'au moins deux ou tous les panneaux de fermeture sont de la même taille et de la même forme les unes par rapport aux autres.

4. Appareil de réchauffement d'air forcé (1) selon l'une quelconque revendication précédente, dans lequel la partie de fermeture (2, 2a, 2b) est partiellement détachable pour ouvrir l'orifice mais reste attachée, par exemple par un raccord cassable.

5. Appareil de réchauffement d'air forcé (1) selon l'une quelconque revendication précédente, dans lequel les panneaux de fermeture d'orifice (6, 6a, 6b) sont plans.

6. Appareil de réchauffement d'air forcé (1) selon l'une quelconque revendication précédente, dans lequel le périmètre de la partie de fermeture (2, 2a, 2b) est au moins partiellement défini par une ligne de division entre la partie de fermeture et la partie de cadre environnante (4, 4a, 4b), de sorte que la partie de fermeture peut être au moins partiellement retirée de la partie de cadre, facultativement dans lequel la ligne de division est une ligne de coupe, ou une ligne de faiblesse ou une ligne de perforations.

7. Appareil de réchauffement d'air forcé (1) selon la revendication 6, dans lequel la ligne de division s'étend partiellement mais pas entièrement autour de la périphérie de la partie de fermeture (2, 2a, 2b) et le panneau de fermeture (6, 6a, 6b) comprend une partie charnière (8, 8a, 8b), la partie charnière reliant la partie de fermeture et la partie cadre.

8. Appareil de réchauffement d'air forcé (1) selon la revendication 7, dans lequel la partie charnière (8, 8a, 8) est pliable de telle sorte que la partie de fermeture (2, 2a, 2b) peut être pliée à l'écart de la partie cadre (4, 4a, 4b) par l'intermédiaire de la partie charnière pliable pour permettre l'utilisation de l'orifice tandis que la partie de fermeture reste reliée au panneau de fermeture, facultativement, dans lequel la partie charnière est déchirable et la partie de fermeture est fixée de manière déchirable à la partie cadre et/ou peut être retirée de manière déchirable de celle-ci.

9. Appareil de réchauffement d'air forcé (1) selon l'une quelconque revendication précédente, dans lequel au moins une partie du matériau qui forme une paroi de l'appareil à air forcé (1) s'étend à l'intérieur de la partie de cadre (4, 4a, 4b).

10. Appareil de réchauffement d'air forcé (1) selon l'une quelconque revendication précédente, dans lequel l'appareil de réchauffement d'air forcé comprend une chambre (16a, 16b) avec deux orifices, ou deux chambres séparées ou plus, chacune ayant un orifice.

11. Appareil de réchauffement d'air forcé (1) selon l'une quelconque revendication précédente, qui comprend deux feuilles de matériau souple qui sont reliées l'une à l'autre par des liaisons (5a, 5b, 10), dans lequel la ou les chambres (16a, 16b) peuvent être définies par des liaisons entre les feuilles, dans lequel une feuille est perméable à l'air et l'autre feuille est imperméable à l'air, et au moins une partie du matériau qui forme la paroi de l'appareil sur lequel est situé un orifice s'étend à l'intérieur de la partie de cadre (4, 4a, 4b).

12. Procédé de fabrication d'un appareil de réchauffement d'air forcé (1) comprenant la mise en contact d'une première feuille (12) d'un matériau imperméable à l'air et d'une seconde feuille d'un matériau perméable à l'air (14) ; la formation d'un ou des joints d'étanchéité (51, 5b, 10) entre la première feuille et la seconde feuille pour définir une ou des chambres (16a, 16b) à l'intérieur de celle-ci ; la formation d'au moins deux orifices dans la première feuille ou dans la seconde feuille ; et la fixation d'un ou des panneaux de fermeture d'orifice (6, 6a, 6b) à la première feuille ou à la seconde feuille de telle sorte que le ou chaque panneau de fermeture d'orifice couvre le ou chaque orifice, dans lequel chaque panneau de fermeture d'orifice (6, 6a, 6b) comprend une partie de cadre (4, 4a, 4b) et une partie de fermeture (2, 2a, 2b), la partie de fermeture (2, 2a, 2b) étant retenue à l'intérieur de la partie de cadre (4, 4a, 4b), dans lequel la partie de fermeture (2, 2a, 2b) obstrue au moins partiellement l'orifice, limitant ainsi l'écoulement d'air à travers l'orifice, dans lequel la partie de fermeture (2, 2a, 2b) peut être au moins partiellement retirée de l'orifice pour permettre l'écoulement d'air à travers l'orifice, dans lequel la partie de fermeture (2, 2a, 2b) peut être partiellement amovible de l'orifice tout en restant fixée à la partie de cadre (4, 4a, 4b) de telle sorte que l'orifice respectif peut être utilisé tandis que la partie de fermeture reste fixée, **caractérisé en ce que** la partie de fermeture (2, 2a, 2b) peut être retirée du panneau de fermeture respectif et peut être fixée à un panneau de fermeture différent, pour obstruer de ce fait l'orifice des différents panneaux de fermeture, et
dans lequel la partie de fermeture (2, 2a, 2b) comprend au moins trois couches de partie de fermeture, les trois couches de partie de fermeture comprenant une première couche de partie de fermeture (26), une couche de partie de fermeture adhésive faisant face à l'orifice (28), et une couche de libération de partie de fermeture protégeant l'adhésif (30).
